(19) Europäisches Patentamt / European Patent Office / Office européen des brevets

(11) EP 3 079 677 B1

(12) FASCICULE DE BREVET EUROPEEN

(45) Date de publication et mention de la délivrance du brevet:
**25.10.2017 Bulletin 2017/43**

(21) Numéro de dépôt: **14827507.6**

(22) Date de dépôt: **12.12.2014**

(51) Int Cl.:
***A61K 9/70*** *(2006.01)* ***C08J 5/18*** *(2006.01)*
***C08G 59/02*** *(2006.01)*

(86) Numéro de dépôt international:
**PCT/FR2014/053318**

(87) Numéro de publication internationale:
**WO 2015/087020 (18.06.2015 Gazette 2015/24)**

(54) **COMPOSITION LIQUIDE PHOTOPOLYMERISABLE**

FLÜSSIGE FOTOPOLYMERISIERBARE ZUSAMMENSETZUNG

PHOTOPOLYMERIZABLE LIQUID COMPOSITION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.12.2013 FR 1362568**

(43) Date de publication de la demande:
**19.10.2016 Bulletin 2016/42**

(73) Titulaires:
- **Urgo Recherche Innovation et Développement 21300 Chenove (FR)**
- **Université De Reims Champagne-Ardenne 51097 Reims Cedex (FR)**
- **Centre National de la Recherche Scientifique (C.N.R.S.) 75794 Paris Cedex 16 (FR)**

(72) Inventeurs:
- **COQUERET, Xavier F-51100 Reims (FR)**
- **DAS, Sadananda Medinipur West Bengal 721168 (IN)**
- **AUGUSTE, Stéphane F-21490 Ruffey les Echirey (FR)**

(74) Mandataire: **Cabinet Plasseraud 66, rue de la Chaussée d'Antin 75440 Paris Cedex 09 (FR)**

(56) Documents cités:
**FR-A1- 2 972 928** **JP-A- 2001 040 068**
**JP-A- 2007 126 612** **US-A- 5 849 460**

- **DATABASE WPI 15 octobre 1986 (1986-10-15) Thomson Scientific, London, GB; AN 1986-314219 XP002728939, TAKAHASHI E; YAMASE Y: "Photocurable epoxy! resin composition - contg. acrylic ester and/or methacrylic ester and sensitiser with polymerisable substit.", & JP JPS6 1231 022 A ((NIPS ) NIPPON SODA CO) 15 octobre 1986 (1986-10-15) & JP S61 231022 A (NIPPON SODA CO) 15 octobre 1986 (1986-10-15)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet une composition liquide photopolymérisable comprenant au moins un pré-polymère polybutadiène époxydé, au moins un photo-amorceur cationique, et au moins un photo-sensibilisateur dérivé de la thioxanthone, de la phénothiazine, de la fluoflavine, de l'anthracène, de la curcumine, du dithienothiophène, des colorants à base de xanthène ou de fluorone, de préférence un dérivé de la thioxanthone pour son utilisation par application sur les tissus tels que la peau, les phanères (les ongles ou les cheveux) et/ou les muqueuses.

Les compositions filmogènes sont destinées à être appliquées, avec ou sans applicateur, sur des tissus tels que la peau, les phanères, les muqueuses. Elles peuvent également être utilisées comme pansement liquide, et sont alors appliquées sur des plaies, des lésions, des cicatrices, des tissus brûlés et/ou affections cutanées. Elles sont généralement liquides à l'application et contiennent classiquement un polymère filmogène dissous dans un solvant volatil, typiquement de l'eau ou un alcool. L'évaporation du solvant permet alors la formation d'un film solide protecteur.

Cependant, les compositions filmogènes à base d'eau ont l'inconvénient de présenter un temps de séchage assez long, et ce d'autant plus long que l'épaisseur du film souhaité est importante. Les compositions filmogènes à base d'alcool ou d'autres solvants organiques peuvent permettre d'obtenir des temps de séchage significativement réduits, mais peuvent être douloureuses lorsqu'elles sont appliquées sur des plaies ou des lésions, et sont ainsi peu adaptées à un usage de type pansement.

En outre, les films solidifiés formés au moyen de ces compositions à base de polymères filmogènes présentent le plus souvent des propriétés mécaniques limitées, notamment en termes d'élasticité et de résistance à la rupture.

Des compositions filmogènes ont été formulées et testées dans le domaine de l'électronique mais leur composition ou leur mise en oeuvre ne sont pas adaptées à des applications sur les tissus tels que la peau, les phanères ou les muqueuses. Ainsi, les compositions décrites dans les demandes de brevet JP 2001 040068 et US 5 849 460 forment des films polymérisés au moyen de lampes à mercure incompatibles avec une application sur des tissus, en particulier la peau. Les demandes JP S61 231022 et JP S61 213217 décrivent des compositions comprenant du Bisphénol A qui est connu pour sa toxicité. Enfin la composition décrite dans la demande JP 2007 126612 met en oeuvre des monomères dont l'application sur des tissus, en particulier sur une peau lésée, est douloureuse et inadaptée à une utilisation comme pansement. FR2972928 divulgue une composition fluide destinée à être appliquée sur la peau, contenant un dérivé de cellulose et une huile végétale. Il existe donc un besoin pour de nouvelles compositions filmogènes non cytotoxiques, capables de former *in-situ* et rapidement des films solides résistants à la rupture, présentant de bonnes propriétés d'adhérence sur les tissus tels que la peau, les phanères ou les muqueuses, et de bonnes propriétés d'élasticité.

La Demanderesse a trouvé, de manière surprenante, qu'il était possible de former de tels films sur tissus tels que la peau, les phanères ou les muqueuses, présentant d'excellentes propriétés de résistance à la rupture, d'adhérence, et d'élasticité, au moyen d'une composition liquide photopolymérisable particulière.

L'invention a ainsi pour objet, selon un premier aspect, une composition liquide photopolymérisable comprenant au moins un pré-polymère polybutadiène époxydé, au moins un photo-amorceur cationique, et au moins un photo-sensibilisateur dérivé de la thioxanthone, de la phénothiazine, de la fluoflavine, de l'anthracène, de la curcumine, du dithienothiophène, des colorants à base de xanthène ou de fluorone, de préférence un dérivé de la thioxanthone, pour son utilisation par application sur les tissus tels que la peau, les phanères et/ou les muqueuses.

Selon un second aspect, l'invention a également pour objet la composition pour son utilisation dans un procédé d'obtention d'un film polymérisé sur les tissus tels que la peau, les phanères ou les muqueuses à partir de ladite composition liquide photopolymérisable.

Une description plus détaillée de certains modes de réalisation préférentiels de l'invention est donnée ci-dessous.

## Description Détaillée de l'Invention

Selon un premier aspect, la présente invention porte sur une composition liquide photopolymérisable comprenant :

- au moins un pré-polymère polybutadiène époxydé,
- au moins un photo-amorceur cationique, et
- au moins un photo-sensibilisateur dérivé de la thioxanthone, de la phénothiazine, de la fluoflavine, de l'anthracène, de la curcumine, du dithienothiophène, de préférence un dérivé de la thioxanthone, pour son utilisation par application sur les tissus tels que la peau, les phanères et/ou les muqueuses.

L'invention a également pour objet la composition décrite précédemment pour son utilisation dans un procédé d'obtention d'un film polymérisé sur les tissus tels que la peau, les phanères et/ou les muqueuses, à partir de la composition liquide photopolymérisable précédemment décrite, caractérisé en que :

i. on applique ladite composition sur les tissus tels que la peau, les phanères ou les muqueuses, de manière à former un film uniforme,
ii. on soumet le film obtenu à l'étape i. à un rayonnement ultraviolet (UV), de préférence un rayonnement UV-visible de longueur d'onde comprise entre 380 et 405 nm, de préférence entre 385 et 395 nm.

En particulier, la composition liquide photopolymérisable selon l'invention est physiologiquement acceptable, c'est à dire non toxique et susceptible d'être appliquée sur les tissus tels que la peau, les phanères ou les muqueuses d'êtres humains ou d'animaux et permet la formation d'un film polymérisé biocompatible.

Pré-polymère polybutadiène époxydé

La composition liquide photopolymérisable selon l'invention comprend au moins un pré-polymère polybutadiène époxydé. Le pré-polymère polybutadiène époxydé est un polybutadiène liquide de faible poids moléculaire, fonctionnalisé par au moins un groupement époxyde polymérisable par voie cationique.

En effet, la Demanderesse a mis en évidence qu'en utilisant un pré-polymère polybutadiène époxydé plutôt que les monomères époxydes conventionnels de faible poids moléculaire, la composition liquide photopolymérisable selon l'invention pouvait être utilisée sur des plaies ouvertes sans générer de sensation douloureuse indésirable (brulure, picotements...) lors de son application. L'utilisation de ce pré-polymère permet par ailleurs d'éviter la diffusion de monomères dans les plaies lors de l'application de la composition sur des lésions, des tissus brûlés et/ou affections cutanées.

Selon un mode de réalisation particulier, le pré-polymère polybutadiène époxydé présente au moins l'une des propriétés suivantes :

- une masse moléculaire comprise entre 500 et 10000 g/mol préférentiellement comprise entre 1000 et 5000 g/mol, et plus préférentiellement comprise entre 1200 et 2500 g/mol,
- une viscosité dynamique à 25°C (298K) comprise entre 0,1 et 100 Pa.s, préférentiellement comprise entre 2,5 et 50 Pa.s, et plus préférentiellement comprise entre 5 et 25 Pa.s, et
- un indice d'époxyde compris entre 0,05 et 1 mol/100g, préférentiellement compris entre 0,1 et 0,5 mol/100g, et plus préférentiellement compris entre 0,15 et 0,45 mol/100g.

L'indice d'époxyde est le nombre de moles de groupements époxydes pour 100 grammes de pré-polymère. L'indice d'époxyde peut notamment être mesuré selon la norme NF EN ISO 3001 (mai 1999, Plastiques - Compositions époxydiques - Détermination de l'équivalent époxy).

Des pré-polymères polybutadiènes époxydés pouvant convenir dans le cadre de la présente demande sont notamment les produits commercialisés par la société Cray Valley sous la dénomination Poly BD605E et Poly BD600E.

Selon un mode de réalisation particulier, le pré-polymère polybutadiène époxydé est présent en une teneur comprise entre 5 et 99,8% en poids, préférentiellement comprise entre 10 et 90% en poids, encore plus préférentiellement entre 15 et 80% en poids, par rapport au poids total de la composition liquide photopolymérisable.

Photo-amorceur cationique

La composition liquide photopolymérisable selon l'invention comprend également au moins un photo-amorceur cationique.

Selon un mode préféré de réalisation, le photo-amorceur cationique est un sel d'onium sensible à un rayonnement UV ou UV-visible et permet d'amorcer la réaction de photopolymérisation.

Il peut notamment être choisi dans le groupe comprenant les sels de diazonium, d'halonium et notamment d'iodonium, de sulfonium, de sulfoxonium et de selenonium.

Des photo-amorceurs cationiques pouvant convenir dans le cadre de la présente demande sont notamment les sels d'aryldiazonium, les sels d'aryliodonium, les sels de diaryliodonium, les sels d'alkylaryliodonium, les sels d'arylsulfonium, les sels de triarylsulfonium, les sels de diarylbromonium, les sels de triarylselenonium, les sels de thioxanthonium, les sels de triarylsulfoxonium, les sels d'aryloxysulfoxonium, les sels de dialkylacylsulfoxonium, les sels de dialkylphenacylsulfonium et les sels de dialkyl-4-hydroxyphenylsulfonium.

Selon un mode de réalisation particulier, le photo-amorceur cationique est choisi parmi le bis(4-tert-butylphényl)iodonium perfluoro-1-butanesulfonate, le bis(4-tert-butylphényl)iodonium p-toluènesulfonate, le bis(4-tert-butylphényl)iodonium triflate, le boc-méthoxyphényldiphénylsulfonium triflate, le (4-bromophényl)diphénylsulfonium triflate, le (tert-butoxycarbonylméthoxynaphthyl)-diphénylsulfonium triflate, le (4-tert-butylphényl)diphénylsulfonium triflate, le diphényliodonium 9,10-diméthoxyanthracène-2-sulfonate, le (p-isopropylphényl)(m-méthyphényl)iodonium tetrakis(pentafluorophenyl)borate, le diphényliodonium hexafluorophosphate, le diphényliodonium nitrate, le diphenyliodonium perfluoro-1-butanesulfonate, le diphényliodonium p-toluènesulfonate, le diphényliodonium triflate, le (4-fluorophényl)diphénylsulfonium triflate, le N-hydroxynaphthalimide triflate, le N-hydroxy-5-norbornène-2,3-dicarboximide perfluoro-1-butanesulfonate, le (4-iodophenyl)diphénylsulfonium, le (4-méthoxyphényl)diphénylsulfonium triflate, le 2-(4-méthoxystyryl)-4,6-bis(trichlorométhyl)-1,3,5-triazin, le (4-méthylphényl)diphénylsulfonium, le (4-méthylthiophényl)méthyl phényl sulfonium triflate, le 1-naphthyl diphénylsulfonium triflate, le (4-phénoxyphényl)diphénylsulfonium triflate, le (4-phénylthiophényl)diphénylsulfonium triflate, les sels de triarylsulfonium hexafluoroantimonate, les sels de triarylsulfonium hexafluorophosphate, le triphénylsulfonium perfluoro-1-butanesufonate, le triphénylsulfonium triflate, le tris(4-tert-butylphényl)sulfonium perfluoro-1-butanesulfonate, le tris(4-tert-butylphényl)sulfonium triflate, le 4-(1-méthyléthyl) phényl 4-méthylphényliodonium tetrakis(pentafluorophenyl) borate (ou diaryl iodonium tetrafluoroborate), les hexafluoroarsenates et leurs mélanges.

Selon un mode de réalisation préférentiel, le photo-amorceur cationique est choisi parmi les sels de diaryliodonium, et est de préférence le 4-(1-methylethyl) phényl 4-methylphenyliodonium tetrakis(pentafluorophenyl) borate (ou diaryl iodonium tetrafluoroborate).

Selon un mode de réalisation particulier, le photo-amorceur cationique est présent en une teneur comprise entre 0,1 et 10% en poids, préférentiellement entre 0,25 et 5% en poids, encore plus préférentiellement entre 0,5 et 1,5% en poids, par rapport au poids total de la composition liquide photopolymérisable.

Photo-sensibilisateur dérivé de la thioxanthone, de la phénothiazine, de la fluoflavine, de l'anthracène, de la curcumine, du dithienothiophène, des colorants à base de xanthène ou de fluorone.

La composition liquide photopolymérisable selon l'invention comprend également au moins un photo-sensibilisateur dérivé de la thioxanthone, de la phénothiazine, de la fluoflavine, de l'anthracène, de la curcumine, du dithienothiophène, des colorants à base de xanthène ou de fluorone.

On entend par photo-sensibilisateur au sens de la présente demande, un composé qui passe à l'état excité par exposition à un rayonnement UV, visible, ou UV-visible, de préférence UV-visible ou visible, et interagit directement avec le photo-amorceur cationique par transfert d'énergie ou d'électron, ou encore par voie indirecte en générant *insitu* des entités radicalaires conduisant à une réduction du photo-amorceur cationique, l'ensemble de ces processus libérant des entités cationiques ou des protons responsables de l'amorçage de la polymérisation cationique des époxydes.

Lorsqu'il est introduit dans les compositions liquides photopolymérisables selon l'invention, le photo-sensibilisateur dérivé de la thioxanthone, de la phénothiazine, de la fluoflavine, de l'anthracène, de la curcumine, du dithienothiophène, des colorants à base de xanthène ou de fluorone confère à la composition une propriété de photoblanchiment, c'est-à-dire qu'il permet au rayonnement UV, visible ou UV-visible de pénétrer sur toute l'épaisseur de la composition liquide à polymériser, favorisant ainsi l'amorçage rapide de la photopolymérisation en profondeur de la composition selon l'invention. Le film obtenu est ainsi polymérisé sur toute son épaisseur de manière uniforme ce qui lui confère d'excellentes propriétés de résistance à la rupture, d'adhérence, et d'élasticité.

Selon un mode particulièrement préféré de réalisation, le photo-sensibilisateur dérivé de la thioxanthone, de la phénothiazine, de la fluoflavine, de l'anthracène, de la curcumine, du dithienothiophène, des colorants à base de xanthène ou de fluorone est sensible à un rayonnement UV-visible dont la longueur d'onde est comprise entre 200 et 500 nm, de préférence entre 300 et 450 nm, et encore plus préférentiellement entre 380 et 405 nm.

Cette plage préférée de longueurs d'ondes permet avantageusement de ne pas endommager les tissus tels que la peau, les phanères ou les muqueuses sur lesquelles la composition liquide photopolymérisable est appliquée, lors de la photopolymérisation.

Selon un mode de réalisation particulier, le photo-sensibilisateur dérivé de la thioxanthone, de la phénothiazine, de la fluoflavine, de l'anthracène, de la curcumine, du dithienothiophène, des colorants à base de xanthène ou de fluorone est présent en une teneur comprise entre 0,1 et 10% en poids, préférentiellement entre 0,25 et 5% en poids, plus préférentiellement entre 0,5 et 1,5% en poids, par rapport au poids total de la composition liquide photopolymérisable.

Selon un autre mode de réalisation particulier, le rapport entre la teneur en photo-sensibilisateur dérivé de la thioxanthone, de la phénothiazine, de la fluoflavine, de l'anthracène, de la curcumine, du dithienothiophène, des colorants à base de xanthène ou de fluorone et la teneur en photo-amorceur cationique dans la composition liquide photopolymérisable de l'invention est compris entre 0,1 et 1, préférentiellement entre 0,5 et 0,75, plus préférentiellement entre 0,6 et 0,7.

Une large gamme de colorants à base de xanthène ou de fluorone peut être utilisée dans la présente invention. Quelques exemples incluent le bleu de méthylène, la rhodamine B, le Rose Bengale, la 3-hydroxy-2,4,5,7-tétraiodo-6-fluorone,5,7-diiodo-3-butoxy-6-fluorone, l'érythrosine B, l'éosine B, érythrosine d'éthyle, l'orange d'acridine, 6'-acétyl-4 ,5,6,7-tétrachloro-2 ',4',5',6',7'-tétraiodofluorescéine (RBAX).

Le photo-sensibilisateur est de préférence un dérivé de la thioxanthone.

Le photo-sensibilisateur dérivé de la thioxanthone est un composé de formule (I) :

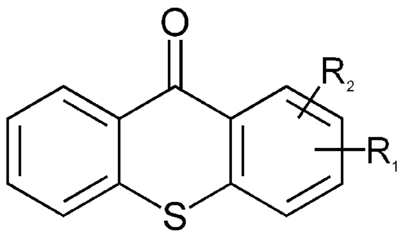

(I)

dans laquelle $R_1$ et $R_2$ sont, indépendamment l'un de l'autre, choisis dans le groupe constitué par un hydrogène, un halogène, et une chaîne carbonée linéaire ou ramifiée, substituée ou non, comprenant de deux à vingt-deux atomes de carbone, qui peut être interrompue par un ou plusieurs atomes d'oxygène, et peut inclure des liaisons carbones saturées ou insaturées.

Les groupes $R_1$ et $R_2$ peuvent inclure, par exemple, les groupes alkyle, alcényl, alcynyle, alcoxy, alcénoxy, alcynoxy et des groupes similaires, linéaires ou ramifiés, pouvant être fonctionnalisés avec des groupes hétéroatomiques ou des radicaux hydrocarbonés porteurs de fonctions hétéroatomiques incluant les groupements hydroxy, nitro, thioéther ou d'autres groupements fonctionnels pharmaceutiquement ou cosmétiquement acceptables.

Selon un mode de réalisation particulier, le photo-sensibilisateur dérivé de la thioxanthone est choisi parmi la 1-chloro-4-propoxythioxanthone, la 2-chlorothioxanthone, la 2,4-diéthylthioxanthone, l'isopropylthioxanthone, la thioxanthone, et leurs mélanges.

Selon un mode de réalisation préférentiel, le photo-sensibilisateur dérivé de la thioxanthone est l'isopropylthioxanthone.

Second pré-polymère différent du polybutadiène époxydé

La composition liquide photopolymérisable selon l'invention peut également comprendre un second pré-polymère, différent du polybutadiène époxydé.

L'ajout d'un second pré-polymère permet notamment d'ajuster la viscosité de la composition à base de polybutadiène époxydé et/ou les propriétés physico-chimiques du film une fois polymérisé, par exemple ses propriétés d'élasticité et d'adhérence sur les tissus tels que la peau, les phanères ou les muqueuses.

Le second pré-polymère est de préférence une huile végétale d'origine naturelle fonctionnalisée par au moins un groupement époxyde polymérisable par voie cationique.

Selon un mode de réalisation particulier, le second pré-polymère est choisi parmi l'huile d'amande époxydée, l'huile d'arachide époxydée, l'huile d'argan époxydée, l'huile de colza époxydée, l'huile de coprah, époxydée, l'huile de lin époxydée, l'huile de maïs époxydée, l'huile de moutarde époxydée, l'huile d'olive époxydée, l'huile de palme époxydée, l'huile de pépins de raisin époxydée, l'huile de ricin époxydée, l'huile de sésame époxydée, l'huile de soja époxydée, l'huile de tournesol époxydée et leurs mélanges.

Selon un mode de réalisation préférentiel, le second pré-polymère est de l'huile de soja époxydée.

Le second pré-polymère peut notamment être introduit en une teneur comprise entre 20 et 80% en poids, préférentiellement entre 25 et 60% en poids, plus préférentiellement entre 30 et 50% en poids, par rapport au poids total du pré-polymère de polybutadiène époxydé et du second pré-polymère.

Composé donneur d'hydrogène

La composition liquide photopolymérisable selon l'invention peut également comprendre un composé donneur d'hydrogène. De façon avantageuse, le composé donneur d'hydrogène peut notamment permettre d'augmenter la cinétique de la réaction de photopolymérisation.

Le composé donneur d'hydrogène peut être un alcool primaire, secondaire ou tertiaire, notamment choisi parmi le n-propanol, l'isopropanol, l'alcool benzylique, le t-butanol et leurs mélanges.

La quantité du composé donneur d'hydrogène peut être comprise entre 1 et 10% en poids, préférentiellement entre 2 et 8% en poids, et plus préférentiellement entre 3 et 5% en poids, par rapport au poids total de la composition liquide photopolymérisable.

Additifs

La composition liquide photopolymérisable selon l'invention peut comprendre, en outre, un ou plusieurs additifs,

de préférence non basiques. En effet, les additifs basiques sont susceptibles d'altérer la photopolymérisation de la composition selon l'invention en interagissant avec les centres actifs assurant la propagation de la polymérisation cationique.

Les additifs pouvant être introduits dans la composition liquide photopolymérisable selon l'invention peuvent notamment être choisis parmi les parfums, les arômes, les colorants, les pigments, les agents matifiants, les agents rhéologiques, les conservateurs, les vitamines, les huiles essentielles et les agents actifs, notamment choisis parmi les agents anti-bactériens, les antiseptiques, les anti-viraux, les agents antifongiques, les anti-douleurs, les antiinflammatoires, les agents favorisant la cicatrisation, les agents hydratants, les agents dépigmentants, les agents kératolytiques, les actifs restructurants, les anesthésiques et leurs mélanges.

En particulier, les actifs pouvant être introduits dans la composition selon l'invention peuvent être choisis parmi :

- les anti-bactériens tels que le Polymyxine B, les pénicillines (Amoxycilline), l'acide clavulanique, les tétracyclines, la Minocycline, la chlorotétracycline, les aminoglycosides, l'Amikacine, la Gentamicine, la Néomycine, l'argent et ses sels (Sulfadiazine argentique), les probiotiques ;
- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol ;
- les anti-viraux tels que [Gamma] Aciclovir, le Famciclovir, le Ritonavir ;
- les antifongiques tels que les polyènes, le Nystatin, l'Amphotéricine B, la Natamycine, les imidazolés (Miconazole, Ketoconazole, Clotrimazole, Econazole, Bifonazole, Butoconazole, Fenticonazole, Isoconazole, Oxiconazole, Sertaconazole, Sulconazole, Thiabendazole, Tioconazole), les triazolés (Fluconazole, Itraconazole, Ravuconazole, Posaconazole, Voriconazole), les allylamines, la Terbinafine, [Gamma] Amorolfme, la Naftifme, la Buténafine ;
- la Flucytosine (antimétabolite), la Griséofulvine, la Caspofungine, la Micafungine ; les anti-douleurs tels que le Paracétamol, la Codéine, le Dextropropoxyphène, le Tramadol, la Morphine et ses dérivés, les Corticoïdes et dérivés ;
- les anti-inflammatoires tels que les Glucocorticoïdes, les anti inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique ;
- les actifs favorisant la cicatrisation tels que le Rétinol, la Vitamine A, la Vitamine E, la N-acétyl-hydroxyproline, les extraits de Centella Asiatica, la papaïne, les silicones, les huiles essentielles de thym, de niaouli, de romarin et de sauge, l'acide hyaluronique, les oligosaccharides polysulfatés synthétiques ayant 1 à 4 unités oses tels que le sel de potassium du sucrose octasulfaté, le sel d'argent du sucrose octasulfaté ou le sucralfate, la metformine, l'aspirine, l'Allantoine ; les agents hydratants tels que l'acide hyaluronique, l'urée, le glycérol, les acides gras, modulateurs des aquaporines, les huiles végétales, le chitosan, certains sucres dont le sorbitol les beurres et les cires ;
- les agents dépigmentants tels que l'acide kojique (Kojic Acid SL(R) - Quimasso (Sino Lion)), l'Arbutine (Olevatin(R) - Quimasso (Sino Linon)), le mélange de palmitoylpropyl de sodium et d'extrait de nénuphar blanc (Sepicalm(R) - Seppic), l'undécylénoyl phénylalanine (Sepiwhite(R) - Seppic), l'extrait de réglisse obtenue par fermentation d'Aspergillus et éthoxydiglycol (Gatuline Whitening(R) - Gattefossé), l'acide octadécènedioïque (ODA White(R) - Sederma), l'alpha-arbutin (Alpha- arbutin(R), SACI-CFPA (Pentapharm)), l'extrait aqueux de feuilles Arctophylos Uva, Ursi (Melfade-J(R) - SACI-CFPA (Pentapharm)), le mélange de plante complexe Gigawhite(R) (SACI-CFPA (Alpaflor)), la diacétyl boldine (Lumiskin(R)-Sederma), l'extrait de mandarine du Japon (Melaslow(R)-Sederma), le mélange d'extrait de citron enrichi en acide citrique et d'extrait de concombre (Uninontan(R)U-34-Unipex), le mélange d'extrait de Rumex occidentalis et de vitamine C (Tyrostat(R) 11 - Unipex), des oligopeptides (Mélanostatine 5(R) - Unipex), le dipalmitate kojique (KAD-15(R) - Quimasso (Sino Lion)), le complexe d'origine naturelle Vegewhite(R) de LCW, des extraits de germe de blé (Clariskin(R) II - Silab), Péthyldiamine triacétate (EDTA); les agents kératolytiques tels que l'acide salicylique, le salicylate de zinc, l'acide ascorbique, les acides alpha hydroxylés (acide glycolique, lactique, malique, citrique, tartrique), les extraits d'Erable argenté, de Griottier, de Tamarinier, l'urée, le rétinoïde topique Kératoline(R) (Sederma), les protéases obtenues par fermentation de Bacillus Subtilis, le produit Linked-Papain(R) (SACI-CFPA), la papaïne (enzyme protéolytique issue du fruit de papaye);
- les actifs restructurants (par exemple resctructurants des phanères) tels que les dérivés de silice, la vitamine E, la camomille, le calcium, l'extrait de prêle, le Lipester de soie; les anesthésiques tels que la benzocaïne, la lidocaïne, la dibucaïne, le chlorhydrate de pramoxine, la bupivacaïne, la mepivacaïne, la prilocaïne, l'étidocaïne.

Selon un mode de réalisation particulier, la composition liquide photopolymérisable se présente sous la forme d'un pansement liquide.

En effet, la composition selon l'invention permet d'obtenir un film polymérisé présentant des propriétés particulièrement intéressantes pour un usage de pansement liquide, c'est-à-dire un film qui adhère à la peau pendant plusieurs

heures, qui ne présente pas de collant superficiel et qui résiste à l'eau, notamment à plusieurs lavages de mains.

La présente invention a également pour objet une composition liquide photopolymérisable telle que décrite précédemment comprenant :

- au moins un pré-polymère polybutadiène époxydé,
- au moins un photo-amorceur cationique, et
- au moins un photo-sensibilisateur dérivé de la thioxanthone, de la phénothiazine, de la fluoflavine, de l'anthracène, de la curcumine, du dithienothiophène, de préférence un dérivé de la thioxanthone,

pour son application sur une plaie et/ou la peau lésée.

Selon un autre aspect, l'invention a pour objet la composition décrite précédemment pour son utilisation dans un procédé d'obtention d'un film polymérisé sur les tissus tels que la peau, les phanères et/ou les muqueuses, à partir de la composition liquide photopolymérisable précédemment décrite, caractérisé en ce que :

i. on applique ladite composition sur les tissus tels que la peau, les phanères ou les muqueuses de manière à former un film liquide uniforme,

ii. on soumet le film obtenu à l'étape i. à un rayonnement UV, de préférence un rayonnement UV-visible de longueur d'onde comprise entre 380 et 405 nm, de préférence entre 385 et 395 nm.

Selon un mode de réalisation particulier, le rayonnement UV est réalisé à une longueur d'onde fixe dont la valeur est choisie entre 385 et 395 nm.

De façon avantageuse, le procédé permet l'obtention facile et rapide d'un film polymérisé présentant d'excellentes propriétés de résistance à la rupture, d'adhérence, et d'élasticité. En particulier, le choix d'un pré-polymère polybutadiène époxydé permet l'obtention rapide d'un film polymérisé car aucun effet inhibiteur de la photopolymérisation par l'oxygène n'est subi.

De plus, la plage de longueurs d'ondes choisie permet avantageusement de ne pas endommager les tissus tels que la peau, les phanères ou les muqueuses sur lesquelles la composition liquide photopolymérisable est appliquée, lors de la photopolymérisation.

Selon un mode préféré de réalisation, le film uniforme formé à l'étape i. présente une épaisseur supérieure à 500 nm, de préférence comprise entre 1 et 300 μm, plus préférentiellement comprise entre 2 et 200 μm.

Selon un mode de réalisation particulier, le film obtenu à l'étape i. est soumis à un rayonnement UV, de préférence UV-visible pendant une durée suffisante pour obtenir une conversion des groupements époxydes d'au moins 50%, de préférence d'au moins 75% En effet, les films polymérisés obtenus à partir des compositions selon l'invention présentent les propriétés physico-chimiques souhaitées lorsque la conversion des groupements époxydes est d'au moins 50% et de préférence d'au moins 75%. La conversion des groupements époxydes est établie à partir d'expérience de spectroscopie IRTF. Elle est calculée en mesurant la hauteur initiale ($h_0$) et la hauteur à chaque instant ($h_t$) des pics d'absorption caractéristiques des groupements époxydes et en appliquant la formule ci-dessous :

$$\text{conversion} = 1-(h_t/h_0)$$

Le film obtenu à l'étape i. est ainsi soumis à un rayonnement UV, de préférence UV-visible pendant une durée comprise entre 5 secondes et 60 minutes, préférentiellement entre 5 secondes et 30 minutes, plus préférentiellement entre 5 secondes et 10 minutes. Ces plages de temps d'exposition permettent avantageusement de ne pas endommager les tissus tels que la peau, les phanères ou les muqueuses sur lesquelles la composition liquide photopolymérisable est appliquée.

Selon un mode de réalisation particulier, l'exposition au rayonnement UV, de préférence UV-visible est réalisée de façon continue.

Selon un mode de réalisation particulier, l'exposition au rayonnement UV, de préférence UV-visible est réalisée de façon discontinue.

La présente invention est illustrée plus en détails dans les exemples non limitatifs décrits ci-après.

**Exemples**

Exemple 1

*Préparation d'une composition liquide photopolymérisable*

On a préparé une composition liquide photopolymérisable comprenant les composés décrits dans le tableau ci-dessous :

| Composé | (% en poids.) |
|---|---|
| Pré-polymère polybutadiène époxydé (Poly BD605E) | 97,5 |
| Diaryl iodonium tetrakis(pentafluorophenyl)borate (Bluesil PI2074) | 1 |
| Isopropylthioxanthone (ITX) | 1,5 |

Le pré-polymère polybutadiène époxydé (Poly BD605E) est commercialisé par Sartomer.
Le diaryl iodonium borate (Bluesil PI2074) est du 4-(1-methylethyl) phenyl 4-methylphenyliodonium tetrakis(pentafluorophenyl) borate commercialisé par BlueStar.
L'isopropylthioxanthone (ITX) utilisé est commercialisée par Aldrich.
On a mélangé tous les composés jusqu'à obtenir une solution homogène. La solution ainsi obtenue a ensuite été conservée à température ambiante et a été protégée de la lumière jusqu'à son utilisation.

*Etude* de *la conversion des groupements époxydes* de *la composition liquide photopolymérisable préparée*

La conversion des groupements époxydes de la composition liquide photopolymérisable est suivie par spectroscopie IFTR (Spectromètre Vertex 70, Bruker). Son évolution est suivie de façon continue en contrôlant la disparition du pic d'absorption caractéristique du groupement époxyde du Poly BD605E à 890 $cm^{-1}$.
La conversion des groupements époxydes de la composition est calculée comme décrit précédemment.
La composition liquide photopolymérisable est préparée selon le protocole suivant. Un dépôt de ladite composition est réalisé sur une plaque de silicium, fixée à un support en aluminium. L'épaisseur du film liquide déposé est de 20 $\mu$m.
La composition est ensuite exposée, sous air et à température ambiante, à un rayonnement UV, de préférence UV-visible, de longueur d'onde de 385 nm émis par une LED-LC3 (Hamamatsu Photonics K.K.) positionnée à une distance de 7,5 cm de la composition et avec une densité de puissance surfacique de 0,1603 $W.cm^{-2}$. L'exposition est réalisée de façon continue pendant une durée de 50 secondes.
La Figure présente l'évolution de la conversion des groupements époxydes de la composition (en %) en fonction du temps d'exposition.
Dans les conditions de l'expérience, après 50 secondes d'exposition, la conversion des groupements époxydes est supérieure à 81%. Le film ainsi obtenu est élastique, adhère à la peau, ne présente pas de collant superficiel, résiste à l'eau, et présente une bonne résistance à la rupture.

Exemple 2

*Etude de l'ajout d'un second pré-polymère à une composition liquide photopolymérisable.*

On a préparé, comme dans l'exemple 1, une composition liquide photopolymérisable comprenant les composés décrits dans le tableau ci-dessous.

| Composé | % massique |
|---|---|
| Poly BD605E | 58,5 |
| Huile de soja époxydée (ESO) | 39 |
| Bluesil PI2074 | 1 |
| ITX | 1,5 |

L'huile de soja époxydée (ESO) est un second pré-polymère commercialisé par Ackross.

La conversion des groupements époxydes de cette composition a été étudiée selon un protocole équivalent à celui décrit dans l'exemple 1.

Cependant la disparition du pic d'absorption caractéristique du groupement époxyde de l'huile de soja époxydée est suivie à 823 $cm^{-1}$. De plus, la durée d'exposition est de 600 secondes.

Dans les conditions de l'expérience, après 600 secondes d'exposition, la conversion des groupements époxydes est supérieure à 80%.

Il apparait que l'ajout d'huile de soja époxydée permet d'obtenir un film ayant des propriétés d'élasticité et d'adhérence à la peau encore améliorées par rapport à celles de la composition de l'exemple 1.

De plus, comme pour le film obtenu avec la composition de l'exemple 1, le film obtenu avec la composition de l'exemple 2 ne présente pas de collant superficiel, résiste à l'eau, et présente une bonne résistance à la rupture.

<u>Exemple 3</u>

*Etude de l'ajout d'un composé donneur d'hydrogène à une composition liquide photopolymérisable.*

On a préparé, comme dans l'exemple 1, une composition liquide photopolymérisable comprenant les composés décrits dans le tableau ci-dessous.

| Composé | % massique |
|---|---|
| Poly BD605E | 92,5 |
| Bluesil PI2074 | 1 |
| ITX | 1,5 |
| Alcool Benzylique | 5 |

L'alcool benzylique est ici utilisé comme un composé donneur d'hydrogène, il est commercialisé par Aldrich. Avant son utilisation, il est conservé sur des tamis moléculaire pour réduire la teneur en humidité.

La conversion des groupements époxydes de cette composition a été étudiée selon un protocole identique à celui décrit dans l'exemple 1.

Dans les conditions de l'expérience, après 50 secondes d'exposition, la conversion des groupements époxydes est supérieure à 84%. La polymérisation est donc accélérée par l'ajout de l'alcool benzylique. De plus, comme pour l'exemple 1, le film obtenu avec la composition de l'exemple 3 est élastique, adhère à la peau, ne présente pas de collant superficiel, résiste à l'eau, et présente une bonne résistance à la rupture.

<u>Exemple 4</u>

*Etude de l'effet du photo-sensibilisateur dérivé* de *la thioxanthone sur le phénomène* de *photoblanchiment, à différentes épaisseurs de films photopolymérisables.*

On a préparé une composition liquide photopolymérisable identique à celle de l'exemple 1.

Le photoblanchiment de l'isopropylthioxanthone a été étudié par mesure continue de l'absorbance à 385 nm de la composition préparée dans un spectrophotomètre UV-Visible.

Le protocole suivi est le suivant. Des échantillons, d'épaisseurs différentes (entre 15 et 200 μm), de la composition préparée ont d'abord été placés entre deux verres fins (150 μm). Ils ont ensuite été exposés à un rayonnement UV-visible de longueur d'onde de 385 nm émis par une LED-LC3 (Hamamatsu Photonics K.K.) positionnée à une distance de 6 cm et avec une densité de puissance surfacique de 0,1807 $W.cm^{-2}$. L'exposition est réalisée de façon continue pendant une durée de 20 secondes.

Dans les conditions de l'expérience, et après les 20 secondes d'exposition, le taux d'avancement du photoblanchiment est compris entre 0,95 et 1 pour l'ensemble des échantillons. Le phénomène de photoblanchiment permet l'amorçage en profondeur de la photopolymérisation. Un taux d'avancement du photoblanchiment compris entre 0,95 et 1 obtenu dans les présentes conditions permet donc une polymérisation du film de manière uniforme sur toute son épaisseur.

Exemple 5

On a préparé, comme dans l'exemple 1, une composition liquide photopolymérisable comprenant les composés décrits dans le tableau ci-dessous *(remplacement de l'ITX par la DETX et l'huile de soja époxydée par l'huile de lin époxydée).*

| Composé | % massique |
|---|---|
| Poly BD605E | 58,5 |
| Bluesil PI2074 | 1 |
| 2,4-diethyl-9H-thioxanthen-9-one (Genocure DETX de Rahn) | 1,5 |
| Huile de lin époxydée | 39 |

Exemple 6

On a préparé, comme dans l'exemple 1, une composition liquide photopolymérisable comprenant les composés décrits dans le tableau ci-dessous *(remplacement de l'ITX par la curcumine* et de *l'huile de soja époxydée par l'huile de lin époxydée).*

| Composé | % massique |
|---|---|
| Poly BD605E | 58,5 |
| Bluesil PI2074 | 1 |
| Curcumine | 1,5 |
| Huile de lin époxydée | 39 |

Exemple 7 :

*Test de résistance à la rupture des compositions des exemples 1, 2 et 3*

Les compositions des exemples 1, 2 et 3 sont étalées de façon à présenter une épaisseur de 55 µm sur un film de polyester présentant une face siliconée. Ces compositions sont alors soumises à une réticulation UV-A (longueurs d'ondes comprises entre 320 et 400 nm) à une puissance de 3.166 Watt/$cm^2$ pendant 15 secondes.

Des échantillons de 70 mm de longueur, 15 mm de largeur de film sont découpées.

On décomplexe les échantillons du support sur lequel ils ont été préparés.

Ces films sont alors soumis à une traction à l'aide d'un dynamomètre. On place les films entre les mors du dynamomètre de manière à avoir 50 mm entre chaque mors. On soumet à une force de 100 N à une vitesse de 300 mm/min jusqu'à rupture.

Les résultats de ces tests figurent dans le tableau suivant :

| | Allongement rupture moyen (%) | Force de rupture moyenne (N/cm) |
|---|---|---|
| Formulation selon l'exemple 1 | 11,6 | 13,57 |
| Formulation selon l'exemple 2 | 22,9 | 2,79 |
| Formulation selon l'exemple 3 | 50, 6 | 8,09 |

Exemple 8 :

*Application de la composition liquide photopolymérisable sur la peau.*

Sur une surface de peau propre et sèche de 3 $cm^2$, on a appliqué avec un pinceau la composition liquide photopolymérisable de l'exemple 1 en une seule couche de 150 µm.

La surface de peau recouverte est ensuite exposée, de façon continue, sous air et à température ambiante, à

un rayonnement UV-visible de longueur d'onde de 385 nm émis par une LED-LC3 (Hamamatsu Photonics K.K.) positionnée à une distance de 7,5 cm de la surface de peau et avec une densité de puissance surfacique de 0,1603 $W.cm^{-2}$.

Après 10 minutes d'exposition, la peau est recouverte d'un film polymérisé qui est élastique, qui adhère à la peau pendant plusieurs heures, qui ne présente pas de collant superficiel, qui résiste à l'eau, notamment à plusieurs lavages de mains et qui présente une bonne résistance à la rupture.

Par ailleurs, sur une surface de peau propre et sèche de 3 $cm^2$, on a appliqué avec un pinceau la composition liquide photopolymérisable des exemples 1, 2 et 3 en une seule couche.

La surface de peau recouverte est ensuite exposée, de façon continue, sous air et à température ambiante, à une lampe LED LZ1 présentant une longueur d'onde de 385 nm et une intensité de 200 mA à 2 cm de la peau pendant 2 minutes.

La tenue dans le temps de la formule selon l'exemple 1 était en moyenne de 18 heures, celle de l'exemple 2 de 6 heures et celle de l'exemple 3 de 28 heures.

## Revendications

**1.** Composition liquide photopolymérisable comprenant:

- au moins un pré-polymère polybutadiène époxydé,
- au moins un photo-amorceur cationique, et
- au moins un photo-sensibilisateur dérivé de la thioxanthone, de la phénothiazine, de la fluoflavine, de l'anthracène, de la curcumine, du dithienothiophène, des colorants à base de xanthène ou de fluorone, de préférence un dérivé de la thioxanthone,

pour son utilisation par application sur les tissus tels que la peau, les phanères et/ou les muqueuses.

**2.** Composition selon la revendication 1, **caractérisée en ce que** le pré-polymère polybutadiène époxydé présente une masse moléculaire comprise entre 500 et 10000 g/mol, préférentiellement comprise entre 1000 et 5000 g/mol, et plus préférentiellement comprise entre 1200 et 2500 g/mol et/ou un indice d'époxyde compris entre 0,05 et 1 mol/100g, préférentiellement compris entre 0,1 et 0,5 mol/100g, et plus préférentiellement compris entre 0,15 et 0,45 mol/100g.

**3.** Composition selon l'une quelconque des revendications précédentes, **caractérisée en ce que** le pré-polymère polybutadiène époxydé est présent en une teneur comprise entre 5 et 99,8% en poids, par rapport au poids total de la composition liquide photopolymérisable.

**4.** Composition selon l'une quelconque des revendications précédentes, **caractérisée en ce que** le photo-amorceur cationique est un sel d'onium sensible au rayonnement UV ou UV-visible, notamment choisi parmi le bis(4-tert-butylphényl)iodonium perfluoro-1-butanesulfonate, le bis(4-tert-butylphényl)iodonium p-toluènesulfonate, le bis(4-tert-butylphényl)iodonium triflate, le boc-méthoxyphényldiphénylsulfonium triflate, le (4-bromophényl)diphénylsulfonium triflate, le (tert-butoxycarbonylméthoxynaphthyl)-diphénylsulfonium triflate, le (4-tert-butylphényl)diphénylsulfonium triflate, le diphényliodonium 9,10-diméthoxyanthracène-2-sulfonate, le (p-isopropylphényl)(m-méthyphényl)iodonium tetrakis(pentafluorophenyl)borate, le diphényliodonium hexafluorophosphate, le diphényliodonium nitrate, le diphenyliodonium perfluoro-1-butanesulfonate, le diphényliodonium p-toluènesulfonate, le diphényliodonium triflate, le (4-fluorophényl)diphénylsulfonium triflate, le N-hydroxynaphthalimide triflate, le N-hydroxy-5-norbornène-2,3-dicarboximide perfluoro-1-butanesulfonate, le (4-iodophenyl)diphénylsulfonium, le (4-méthoxyphényl)diphénylsulfonium triflate, le 2-(4-méthoxystyryl)-4,6-bis(trichlorométhyl)-1,3,5-triazin, le (4-méthylphényl)diphénylsulfonium, le (4-méthylthiophényl)méthyl phényl sulfonium triflate, le 1-naphthyl diphénylsulfonium triflate, le (4-phénoxyphényl)diphénylsulfonium triflate, le (4-phénylthiophényl)diphénylsulfonium triflate, les sels de triarylsulfonium hexafluoroantimonate, les sels de triarylsulfonium hexafluorophosphate, le triphénylsulfonium perfluoro-1-butanesufonate, le triphénylsulfonium triflate, le tris(4-tert-butylphényl)sulfonium perfluoro-1-butanesulfonate, le tris(4-tert-butylphényl)sulfonium triflate, le 4-(1-méthyléthyl) phényl 4-méthylphényliodonium tetrakis(pentafluorophenyl) borate (ou diaryl iodonium tetrafluoroborate), les hexafluoroarsenates et leurs mélanges, de préférence le photo-amorceur cationique est choisi parmi les sels de diaryliodonium, et est de préférence le 4-(1-methylethyl) phényl 4-methylphenyliodonium tetrakis(pentafluorophenyl) borate (ou diaryl iodonium tetrafluoroborate).

**5.** Composition selon l'une quelconque des revendications précédentes, **caractérisée en ce que** le photo-amorceur cationique est présent en une teneur comprise entre 0,1 et 10% en poids, par rapport au poids total de la composition

liquide photopolymérisable.

6. Composition selon l'une quelconque des revendications précédentes, **caractérisée en ce que** le photo-sensibilisateur est présent en une teneur comprise entre 0,1 et 10% en poids, par rapport au poids total de la composition liquide photopolymérisable.

7. Composition selon l'une quelconque des revendications précédentes, **caractérisée en ce que** le photo-sensibilisateur est un dérivé de la thioxanthone choisi parmi la 1-chloro-4-propoxythioxanthone, la 2-chlorothioxanthone, la 2,4-diéthylthioxanthone, l'isopropylthioxanthone, la thioxanthone, et leurs mélanges, et de préférence est l'isopropylthioxanthone.

8. Composition selon l'une quelconque des revendications précédentes, **caractérisée en ce qu'**elle comprend un second pré-polymère, différent du polybutadiène époxydé.

9. Composition selon la revendication précédente, **caractérisée en ce que** le second pré-polymère est choisi parmi l'huile d'amande époxydée, l'huile d'arachide époxydée, l'huile d'argan époxydée, l'huile de colza époxydée, l'huile de coprah, époxydée, l'huile de lin époxydée, l'huile de maïs époxydée, l'huile de moutarde époxydée, l'huile d'olive époxydée, l'huile de palme époxydée, l'huile de pépins de raisin époxydée, l'huile de ricin époxydée, l'huile de sésame époxydée, l'huile de soja époxydée, l'huile de tournesol époxydée et leurs mélanges, de préférence le second pré-polymère est de l'huile de soja époxydée.

10. Composition selon la revendication 8 ou 9, **caractérisée en ce que** le second pré-polymère est introduit en une teneur comprise entre 20 et 80% en poids, par rapport au poids total du pré-polymère de polybutadiène époxydé et du second pré-polymère.

11. Composition liquide photopolymérisable comprenant:

- au moins un pré-polymère polybutadiène époxydé,
- au moins un photo-amorceur cationique, et
- au moins un photo-sensibilisateur dérivé de la thioxanthone, de la phénothiazine, de la fluoflavine, de l'anthracène, de la curcumine, du dithienothiophène, des colorants à base de xanthène ou de fluorone, de préférence un dérivé de la thioxanthone, pour son utilisation dans un procédé d'obtention d'un film polymérisé sur les tissus tels que la peau, les phanères et/ou les muqueuses, ledit procédé étant caractérisé en que :

  i. on applique ladite composition sur les tissus tels que la peau, les phanères ou les muqueuses de manière à former un film liquide uniforme,
  ii. on soumet le film obtenu à l'étape i. à un rayonnement UV, de préférence un rayonnement UV-visible de longueur d'onde comprise entre 380 et 405 nm, de préférence entre 385 et 395 nm.

12. Composition selon la revendication 11, **caractérisée en ce que** l'épaisseur du film liquide uniforme est supérieure à 500 nm, de préférence comprise entre 1 et 300 $\mu$m, plus préférentiellement comprise entre 2 et 200 $\mu$m.

13. Composition selon la revendication 11 ou 12, **caractérisée en ce que** film obtenu à l'étape i. est soumis à un rayonnement UV, de préférence UV-visible pendant une durée comprise entre 5 secondes et 60 minutes, préférentiellement, entre 5 secondes et 30 minutes, plus préférentiellement entre 5 secondes et 10 minutes.

## Patentansprüche

1. Flüssige photopolymerisierbare Zusammensetzung, umfassend:

- mindestens ein epoxidiertes Polybutadien-Präpolymer,
- mindestens einen kationischen Photoinitiator und
- mindestens einen Photosensibilisator, abgeleitet von Thioxanthon, Phenothiazin, Fluoflavin, Anthracen, Curcumin, Dithienothiophen, Farbstoffen auf Basis von Xanthen oder Fluoron, vorzugsweise ein Derivat von Thioxanthon,

zu seiner Verwendung zur Anwendung auf Gewebe, wie etwa Haut, Adnexe (Hautanhangsgebilde) und/oder

Schleimhautgewebe.

2. Zusammensetzung nach Anspruch 1, **dadurch gekennzeichnet, dass** das epoxidierte Polybutadien-Präpolymer eine Molmasse zwischen 500 und 10000 g/mol umfasst, vorzugsweise zwischen 1000 und 5000 g/mol umfasst und mehr bevorzugt zwischen 1200 und 2500 g/mol umfasst, und/oder einen Epoxidindex zwischen 0,05 und 1 mol/100 g umfasst, vorzugsweise zwischen 0,1 und 0,5 mol/100 g umfasst und mehr bevorzugt zwischen 0,15 und 0,45 mol/ 100 g umfasst.

3. Zusammensetzung nach einem der vorhergehenden Ansprüche, **dadurch gekennzeichnet, dass** das epoxidierte Polybutadien-Präpolymer in einer Menge vorliegt, umfassend zwischen 5 und 99,8 Gew.-% bezüglich des Gesamtgewichts der flüssigen photopolymerisierbaren Zusammensetzung.

4. Zusammensetzung nach einem der vorhergehenden Ansprüche, **dadurch gekennzeichnet, dass** der kationische Photoinitiator ein Oniumsalz ist, das empfindlich auf UV- oder sichtbares UV-Strahlung ist, insbesondere ausgewählt aus Bis(4-tert-butylphenyl)iodoniumperfluor-1-butansulfonat, Bis(4-tert-butylphenyl)iodonium-p-toluolsulfonat, Bis(4-tert-butylphenyl)iodonium-triflat, Boc-methoxyphenyldiphenylsulfonium-triflat, (4-Bromphenyl)diphenylsulfonium-triflat, (tert-Butoxycarbonylmethoxynaphthyl)-diphenylsulfonium-triflat, (4-tert-Butylphenyl)diphenylsulfonium-triflat, Diphenyliodonium-9,10-dimethoxyanthracen-2-sulfonat, (p-Isopropylphenyl)(m-methylphenyl)iodonium-tetrakis(pentafluorphenyl)borat, Diphenyliodoniumhexafluorphosphat, Diphenyliodoniumnitrat, Diphenyliodonium-perfluor-1-butansulfonat, Diphenyliodonium-p-toluolsulfonat, Diphenyliodonium-triflat, (4-Fluorphenyl)diphenylsulfonium-triflat, N-Hydroxynaphthalimid-triflat, N-Hydroxy-5-norbornen-2,3-dicarboximid-perfluor-1-butansulfonat, (4-Iodphenyl)diphenylsulfonium, (4-Methoxyphenyl)diphenylsulfonium-triflat, 2-(4-Methoxystyryl)-4,6-bis(trichlormethyl)-1,3,5-triazin, (4-Methylphenyl)diphenylsulfonium, (4-Methylthiophenyl)methylphenylsulfonium-triflat, 1-Naphthyl-diphenylsulfonium-triflat, (4-Phenoxyphenyl)diphenylsulfonium-triflat, (4-Phenylthiophenyl)diphenylsulfonium-triflat, den Salzen von Triarylsulfonium-hexafluorantimonat, den Salzen von Triarylsulfonium-hexafluorphosphat, Triphenylsulfonium-perfluor-1-butansulfonat, Triphenylsulfonium-triflat, Tris(4-tert-butylphenyl)sulfonium-perfluor-1-butansulfonat, Tris(4-tert-butylphenyl)sulfonium-triflat, 4-(1-Methylethyl)phenyl-4-methylphenyliodonium-tetrakis(pentafluorphenyl)borat (oder Diaryliodoniumtetrafluorborat), den Hexafluorarsenaten und ihren Gemischen, wobei der kationische Photoinitiator vorzugsweise ausgewählt ist aus den Salzen von Diaryliodonium und bevorzugt 4-(1-Methylethyl)phenyl-4-methylphenyliodonium-tetrakis(pentafluorphenyl)borat (oder Diaraliodonium-tetrafluorborat).

5. Zusammensetzung nach einem der vorhergehenden Ansprüche, **dadurch gekennzeichnet, dass** der kationische Photoinitiator in einer Menge von zwischen 0,1 und 10 Gew.-% bezüglich des Gesamtgewichts der flüssigen photopolymerisierbaren Zusammensetzung vorliegt.

6. Zusammensetzung nach einem der vorhergehenden Ansprüche, **dadurch gekennzeichnet, dass** der kationische Photosensibilisator in einer Menge von zwischen 0,1 und 10 Gew.-% bezüglich des Gesamtgewichts der flüssigen photopolymerisierbaren Zusammensetzung vorliegt.

7. Zusammensetzung nach einem der vorhergehenden Ansprüche, **dadurch gekennzeichnet, dass** der Photosensibilisator ein Derivat von Thioxanthon ist, ausgewählt aus 1-Chlor-4-propoxythioxanthon, 2-Chlorthioxanthon, 2,4-Diethylthioxanthon, Isopropylthioxanthon, Thioxanthon und ihren Gemischen, und vorzugsweise Isopropylthioxanthon ist.

8. Zusammensetzung nach einem der vorhergehenden Ansprüche, **dadurch gekennzeichnet, dass** sie ein zweites Präpolymer, das verschieden von epoxidiertem Polybutadien ist, umfasst.

9. Zusammensetzung nach dem vorhergehenden Anspruch, **dadurch gekennzeichnet, dass** das zweite Präpolymer ausgewählt ist aus epoxidiertem Mandelöl, epoxidiertem Erdnussöl, epoxidiertem Arganöl, epoxidiertem Rapsöl, epoxidiertem Kopraöl, epoxidiertem Leinöl, epoxidiertem Maisöl, epoxidiertem Senföl, epoxidiertem Olivenöl, epoxidiertem Palmöl, epoxidiertem Traubenkernöl, epoxidiertem Rizinusöl, epoxidiertem Sesamöl, epoxidiertem Sojaöl, epoxidiertem Sonnenblumenöl und ihren Gemischen, wobei das zweite Präpolymer vorzugsweise epoxidiertes Sojaöl ist.

10. Zusammensetzung nach Anspruch 8 oder 9, **dadurch gekennzeichnet, dass** das zweite Präpolymer in einer Menge zwischen 20 und 80 Gew.-% bezüglich des Gesamtgewichts von Präpolymer aus epoxidiertem Polybutadien und zweitem Präpolymer eingebracht wird.

**11.** Photopolymerisierbare flüssige Zusammensetzung, umfassend:

- mindestens ein epoxidiertes Polybutadien-Präpolymer,
- mindestens einen kationischen Photoinitiator und
- mindestens einen Photosensibilisator, abgeleitet von Thioxanthon, Phenothiazin, Fluoflavin, Anthracen, Curcumin, Dithienothiophen, Farbstoffen auf Basis von Xanthen oder Fluoron, vorzugsweise ein Derivat von Thioxanthon, zu seiner Verwendung in einem Verfahren zum Herstellen eines polymerisierten Films auf Geweben, wie Haut, Adnexen und/oder Schleimhautgeweben, wobei das Verfahren **dadurch gekennzeichnet ist, dass**:

i. die Zusammensetzung auf Geweben, wie Haut, Adnexen und/oder Schleimhautgeweben auf eine solche Art aufgebracht wird, um einen gleichmäßigen flüssigen Film zu bilden,
ii. der aus Schritt i. erhaltene Film einer UV-Bestrahlung ausgesetzt wird, vorzugsweise einer Bestrahlung im sichtbaren UV mit einer Wellenlänge zwischen 380 und 405 nm, vorzugsweise zwischen 385 und 395 nm.

**12.** Zusammensetzung nach Anspruch 11, **dadurch gekennzeichnet, dass** die Dicke des gleichmäßigen flüssigen Films über 500 nm ist, vorzugsweise zwischen 1 und 300 $\mu$m, mehr bevorzugt zwischen 2 und 200 $\mu$m ist.

**13.** Zusammensetzung nach Anspruch 11 oder 12, **dadurch gekennzeichnet, dass** der in Schritt i. erhaltene Film einer UV-Bestrahlung, vorzugsweise sichtbarem UV, für eine Dauer zwischen 5 Sekunden und 60 Minuten, bevorzugt zwischen 5 Sekunden und 30 Minuten, mehr bevorzugt zwischen 5 Sekunden und 10 Minuten ausgesetzt wird.

## Claims

**1.** Photopolymerizable liquid composition comprising:

- at least one epoxidized polybutadiene prepolymer,
- at least one cationic photoinitiator, and
- at least one photosensitizer derived from thioxanthone, phenothiazine, fluoflavin, anthracene, curcumin, dithienothiophene, or xanthene-based or fluorone-based dyes, preferably a thioxanthone derivative,

for its use by application thereof to tissues such as the skin, the skin appendages and/or the mucous membranes.

**2.** The composition as claimed in claim 1, **characterized in that** the epoxidized polybutadiene prepolymer has a molecular weight of between 500 and 10 000 g/mol, preferentially of between 1000 and 5000 g/mol, and more preferentially of between 1200 and 2500 g/mol, and/or an epoxide number of between 0.05 and 1 mol/100 g, preferentially of between 0.1 and 0.5 mol/100 g, and more preferentially of between 0.15 and 0.45 mol/100 g.

**3.** The composition as claimed in either one of the preceding claims, **characterized in that** the epoxidized polybutadiene prepolymer is present in a content of between 5 and 99.8% by weight, relative to the total weight of the photopolymerizable liquid composition.

**4.** The composition as claimed in any one of the preceding claims, **characterized in that** the cationic photoinitiator is an onium salt sensitive to UV or UV-visible radiation, in particular chosen from bis(4-tert-butylphenyl)iodonium perfluoro-1-butanesulfonate, bis(4-tert-butylphenyl)iodonium p-toluenesulfonate, bis(4-tert-butylphenyl)iodonium triflate, boc-methoxyphenyldiphenylsulfonium triflate, (4-bromophenyl)diphenylsulfonium triflate, (tert-butoxycarbonylmethoxynaphthyl)diphenylsulfonium triflate, (4-tert-butylphenyl)diphenylsulfonium triflate, diphenyliodonium 9,10-dimethoxyanthracene-2-sulfonate, (p-isopropylphenyl)(m-methylphenyl)iodonium tetrakis(pentafluorophenyl)borate, diphenyliodonium hexafluorophosphate, diphenyliodonium nitrate, diphenyliodonium perfluoro-1-butanesulfonate, diphenyliodonium p-toluenesulfonate, diphenyliodonium triflate, (4-fluorophenyl)diphenylsulfonium triflate, N-hydroxynaphthalimide triflate, N-hydroxy-5-norbornene-2,3-dicarboximide perfluoro-1-butanesulfonate, (4-iodophenyl)diphenylsulfonium, (4-methoxyphenyl)diphenylsulfonium triflate, 2-(4-methoxystyryl)-4,6-bis(trichloromethyl)-1,3,5-triazine, (4-methylphenyl)diphenylsulfonium, (4-methylthiophenyl)methyl phenyl sulfonium triflate, 1-naphthyl diphenylsulfonium triflate, (4-phenoxyphenyl)diphenylsulfonium triflate, (4-phenylthiophenyl)diphenylsulfonium triflate, triarylsulfonium hexafluoroantimonate salts, triarylsulfonium hexafluorophosphate salts, triphenylsulfonium perfluoro-1-butanesulfonate, triphenylsulfonium triflate, tris(4-tert-butylphenyl)sulfonium perfluoro-1-butanesulfonate, tris(4-tert-butylphenyl)sulfonium triflate, 4-(1-methylethyl) phenyl 4-methylphenyliodonium tetrakis(pentafluorophenyl)borate (or diaryl iodonium tetrafluoroborate), hexafluoroarsenates, and mixtures thereof;

preferably the cationic photoinitiator is chosen from diaryliodonium salts, and is preferably 4-(1-methylethyl) phenyl 4-methylphenyliodonium tetrakis(pentafluorophenyl)borate (or diaryl iodonium tetrafluoroborate).

5. The composition as claimed in any one of the preceding claims, **characterized in that** the cationic photoinitiator is present in a content of between 0.1 and 10% by weight, relative to the total weight of the photopolymerizable liquid composition.

6. The composition as claimed in any one of the preceding claims, **characterized in that** the photosensitizer is present in a content of between 0.1 and 10% by weight, relative to the total weight of the photopolymerizable liquid composition.

7. The composition as claimed in any one of the preceding claims, **characterized in that** the photosensitizer is a thioxanthone derivative chosen from 1-chloro-4-propoxythioxanthone, 2-chlorothioxanthone, 2,4-diethylthioxanthone, isopropylthioxanthone, thioxanthone, and mixtures thereof, and is preferably isopropylthioxanthone.

8. The composition as claimed in any one of the preceding claims, **characterized in that** it comprises a second prepolymer, different than the epoxidized polybutadiene.

9. The composition as claimed in the preceding claim, **characterized in that** the second prepolymer is chosen from epoxidized almond oil, epoxidized peanut oil, epoxidized argan oil, epoxidized rapeseed oil, epoxidized coconut oil, epoxidized linseed oil, epoxidized corn oil, epoxidized mustard oil, epoxidized olive oil, epoxidized palm oil, epoxidized grapeseed oil, epoxidized castor oil, epoxidized sesame oil, epoxidized soybean oil, epoxidized sunflower oil, and mixtures thereof; preferably the second prepolymer is epoxidized soybean oil.

10. The composition as claimed in claim 8 or 9, **characterized in that** the second prepolymer is introduced in a content of between 20% and 80% by weight, relative to the total weight of the epoxidized polybutadiene prepolymer and of the second prepolymer.

11. Photopolymerizable liquid composition comprising:

- at least one epoxidized polybutadiene prepolymer,
- at least one cationic photoinitiator, and
- at least one photosensitizer derived from thioxanthone, phenothiazine, fluoflavin, anthracene, curcumin, dithienothiophene, or xanthene-based or fluorone-based dyes, preferably a thioxanthone derivative, for its use in a process for obtaining a polymerized film on tissues such as the skin, the skin appendages and/or the mucous membranes, said process being **characterized in that**:

    i. said composition is applied to tissues such as the skin, the skin appendages or the mucous membranes so as to form a uniform liquid film,
    ii. the film obtained in step i. is subjected to UV radiation, preferably UV-visible radiation having a wavelength of between 380 and 405 nm, preferably between 385 and 395 nm.

12. The composition as claimed in claim 11, **characterized in that** the thickness of the uniform liquid film is greater than 500 nm, preferably between 1 and 300 μm, more preferentially between 2 and 200 μm.

13. The composition as claimed in claim 11 or 12, **characterized in that** the film obtained in step i. is subjected to UV radiation, preferably UV-visible radiation, for a period of between 5 seconds and 60 minutes, preferentially between 5 seconds and 30 minutes, more preferentially between 5 seconds and 10 minutes.

FIGURE

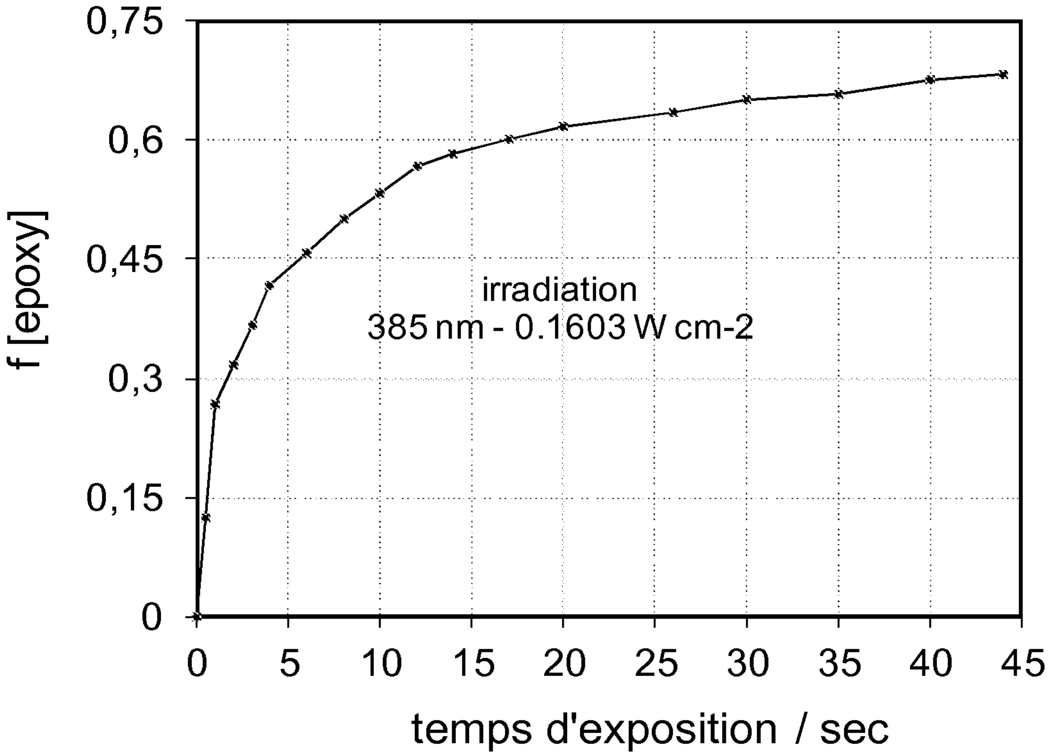
0,75
0,6
0,45
0,3
0,15
0
f [epoxy]
irradiation
385 nm - 0.1603 W cm-2
0
5
10
15
20
25
30
35
40
45
temps d'exposition / sec

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- JP 2001040068 A
- US 5849460 A
- JP S61231022 A
- JP S61213217 A
- JP 2007126612 A
- FR 2972928